# EUROPEAN PATENT APPLICATION

(11) **EP 2 003 110 A2**
(43) Date of publication of application: **17.12.2008**
(21) Application number: 07739276.9
(22) Date of filing: 22.03.2007
(51) Int. Cl.: C07C 41/03, C07C 43/13, C08G 65/28, C11D 1/66

(54) **METHOD FOR PRODUCING (POLY)GLYCERYL ETHER**

(30) Priority: 31.03.2006 JP 2006099681
(71) Applicant: Kao Corporation, Chuo-ku Tokyo 103-8210 (JP)
(72) Inventor: NAGASAWA, Atsushi, Wakayama-shi, Wakayama 640-8580 (JP); SAITO, Akira, Wakayama-shi, Wakayama 640-8580 (JP); UNO, Mitsuru, Wakayama-shi, Wakayama 640-8580 (JP)
(74) Representative: HOFFMANN EITLE
(86) International application number: PCT/JP2007/055833
(87) International publication number: WO 2007/114061

(57) **Abstract**

The present invention relates to a process for producing a (poly)glyceryl ether which includes the step of reacting an alcohol with glycidol under a neutral condition. According to the process of the present invention, the (poly)glyceryl ether having a narrow molecular weight distribution can be produced with a high yield in a simplified manner.

## Description

### Field of the Invention

The present invention relates to a process for producing (poly)glyceryl ethers.

### Background of the Invention

(Poly)glyceryl ethers exhibit excellent properties as a nonionic surfactant, and have been used in various applications such as food, cosmetics, toiletries, agricultural chemicals and drugs for the purposes of emulsification, solubilization, dispersion, cleaning, foaming, defoaming, penetration, antibacterial effect, etc. There are conventionally known the following methods (1) to (6) for producing the (poly)glyceryl ethers.
(1) Method of reacting an alkyl halide with hydroxyl groups of (poly)glycerol. However, this method has such a problem that since a large number of hydroxyl groups present in the (poly)glycerol are subjected to the reaction, one or more alkyl groups tend to be added to the obtained (poly)glyceryl ethers.
(2) Method of reacting an alkyl glycidyl ether with water and polyglycerol to subject an epoxy ring of the alkyl glycidyl ether to ring opening. However, this method has such a problem that one or more alkyl glycidyl ether molecules are reacted.

(3) Method of adding 1 mol of epichlorohydrin to an aliphatic alcohol, subjecting the resultant addition product to dehydrochlorination and ring closure in the presence of an alkali, and then subjecting the resultant ring-closed product again to ring opening using diluted sulfuric acid, followed by repeating these procedures until reaching the aimed polymerization degree. (4) Method of addition-polymerizing a glycidyl ester with an aliphatic alcohol, and then subjecting the resultant addition product to saponification using an alkali to eliminate an acyl group therefrom (refer to Patent Document 1). (5) Method of reacting an alkyl glycidyl ether with glycerol to synthesize an alkyl diglyceryl ether, etherifying hydroxyl groups of the alkyl diglyceryl ether with an allyl halide, and then converting from the allyl group of the resultant compound into two hydroxyl groups, followed by repeating these procedures until reaching the aimed polymerization degree (refer to Patent Document 2). However, these methods have problems such as complicated reaction steps and, therefore, are still unsatisfactory as industrial methods.

(6) Method of addition-polymerizing glycidol with an aliphatic alcohol in the presence of a catalyst (refer to Non-Patent Document 1). However, in this method, when using an acid as the catalyst, a polymerization reaction between glycidol molecules tends to proceed, resulting in production of a large amount of polyglycerol, whereas when using a base as the catalyst, it tends to be difficult to control a molecular weight distribution of the obtained product, resulting in production of such a (poly)glyceryl ether having a broad molecular weight distribution. In order to suppress these undesirable reactions, there is known the method of using the alcohol in a large excessive amount relative to the glycidol. However, this method tends to be considerably deteriorated in productivity, resulting in industrially disadvantageous process.

Patent Document 1: JP 9-188755A
Patent Document 2: JP 2001-114720A
Non-Patent Document 1: "GLYCIDOL; Properties, Reactions, Applications", Kleeman, Axel Dr. Alfred Huthig, Verlag Heidelberg, 1981

### Summary of the Invention

The present invention relates to a process for producing a (poly)glyceryl ethers by reacting an alcohol with glycidol under a neutral condition.

### Detailed Description of the Invention

As described above, the conventional methods for producing (poly)glyceryl ethers are unsatisfactory for producing (poly)glyceryl ether compounds having a narrow molecular weight distribution with a high yield in a simplified manner.
The present invention relates to a process for producing a (poly)glyceryl ether having a narrow molecular weight distribution with a high yield in a simplified manner while suppressing production of by-products such as polymers.
The present inventors have found that the above conventional problems can be solved by reacting an alcohol with glycidol under a neutral condition.
That is, the present invention relates to a process for producing a (poly)glyceryl ether which include the step of reacting an alcohol with glycidol under a neutral condition.

The process for producing a (poly)glyceryl ether according to the present invention is characterized by reacting an alcohol with glycidol under a neutral condition. The (poly)glyceryl ether described herein means a glyceryl ether having one or more glycerol residues in a molecule thereof.
The alcohol used in the present invention is preferably a compound represented by the following general formula (1):

R¹-(OR²)ₙO-H (1)

wherein R¹ is a hydrocarbon group having 4 to 24 carbon atoms; R² is an alkylene group having 2 or 3 carbon atoms; and n represents an average polymerization degree of the oxyalkylene group and is a number of from 0 to 20.

In the general formula (1), R¹ is preferably a linear, branched or cyclic alkyl group or alkenyl group having 6 to 18 carbon atoms.
Specific examples of R¹ include butyl, hexyl, heptyl, octyl, nonyl, decyl, undecyl, dodecyl, myristyl, pentadecyl, palmityl, stearyl, behenyl, 2-ethylhexyl, hexenyl, heptenyl, octenyl, nonenyl, decenyl, undecenyl, dodecenyl, myristenyl, pentadecenyl, palmitenyl, oleyl, linoleyl, linolenyl, arachidyl, 2-ethylhexenyl, cyclopentyl, cyclohexyl and cyclooctyl. Among these groups, preferred are linear or branched alkyl groups having 6 to 14 carbon atoms, more preferred are linear or branched alkyl groups having 6 to 12 carbon atoms, and still more preferred are linear alkyl groups having 6 to 10 carbon atoms.
Specific examples of R² include an ethylene group (-C₂H₄-) and a propylene group (-C₃H₆-).
The suffix n represents an average polymerization degree of the oxyalkylene group (-OR²-) and is preferably a number of from 0 to 8, more preferably from 0 to 6 and most preferably 0.

In the present invention, the alcohol is reacted with glycidol represented by the following formula (2). In the reaction, when using the compound represented by the following general formula (1) as the alcohol, the (poly)glyceryl ether represented by the following general formula (3) is produced as shown in the following reaction formula.

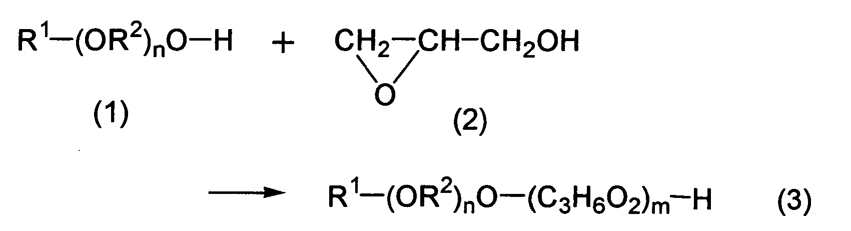

In the general formula (3), R¹, R² and n are the same as defined above, and m represents an average polymerization degree of the glycerol residue and is a number of from 1 to 10. Also, the preferred ranges of R¹, R² and n are the same as described above, and m is preferably a number of from 1 to 5, more preferably from 1 to 4, still more preferably from 1 to 3, and most preferably 1.
The glycerol residue used herein means a group represented by the formula: -CH₂-CH(OH)-CH₂O- and/or a group represented by the formula:

-CH₂-CH(CH₂OH)-O-.

In the present invention, the reaction between the alcohol and glycidol is conducted under a neutral condition. The "neutral condition" used herein means that the pH of the reaction system lies within the range of from 6 to 8, preferably from 6.5 to 7.5 and more preferably from 6.5 to 7.0, but is not limited to only the case where the pH of the reaction system is 7.
The method of conducting the above reaction under a neutral condition is not particularly limited, and there may be used, for example, the method of using high-purity compounds as the raw alcohol and glycidol, or the method of using such raw materials from which acid substances derived from the raw materials are removed. When the acid substances are contained in the raw materials to such an extent that the pH of the reaction system is adversely affected thereby, commercially available pH controllers may be added thereto.
The "pH of the reaction system" used herein means the value determined by mixing a mixed solution prior to adding glycidol thereto with an equal amount of ion-exchanged water to separate a water layer therefrom, and then measuring the pH of the thus separated water layer.
The ratio between amounts of the alcohol and glycidol used is not particularly limited, and is suitably controlled such that the molar ratio of glycidol to the alcohol (glycidol/alcohol) is preferably from 0.001 to 4, more preferably from 0.001 to 1, still more preferably from 0.01 to 0.8, further still more preferably from 0.05 to 0.6, and further still more preferably from 0.2 to 0.5.

In the present invention, a catalyst may or may not be used. The catalyst may be used as long as the reaction system is still kept in a neutral condition even when adding the catalyst thereto. Examples of the catalyst include acid catalysts such as Lewis acids and basic catalysts such as metal alcoholates. The reaction is more preferably conducted in the absence of any catalyst.
More specifically, in the preferred reaction, the alcohol is charged into a reactor, and then glycidol is added to the reactor without adding any catalyst thereto, thereby allowing these compounds to be reacted with each other under a neutral condition.

The reaction between the alcohol and glycidol is an exothermic reaction. Therefore, the reaction is preferably gradually carried out by continuously dropping glycidol to the alcohol or intermittently adding divided parts of glycidol thereto, while stirring the alcohol.
The dropping rate of glycidol is preferably 10% by mass/min or less, more preferably 2% by mass/min or less, still more preferably 1% by mass/min or less, further still more preferably 0.75% by mass/min or less, and further still more preferably 0.5% by mass/min or less on the basis of a whole amount of glycidol to be charged. The lower limit of the dropping rate of glycidol is preferably 0.1% by mass/min or more.
When intermittently adding divided parts or installments of glycidol to the alcohol, the amount of glycidol to be added is equally divided into preferably four or more parts, more preferably 6 or more parts and still more preferably 8 or more parts, and the divided parts or installments of glycidol are added to the alcohol at equal intervals such that the addition rate (dropping rate) thereof falls within the above specified range as a whole.
The time required for continuously dropping glycidol or intermittently adding divided parts of glycidol, varies depending upon the amount of glycidol to be added, etc., and is preferably from 0.25 to 24 h, more preferably from 1 to 10 h and still more preferably from 2 to 5 h from the industrial viewpoints.
In addition, after completing the addition of glycidol, the reaction system may be kept under the same condition over a period of from 0.1 to 3 h for aging thereof.

The reaction temperature is preferably from 100 to 200°C, more preferably from 130 to 180°C and still more preferably from 145 to 165°C from a good reaction efficiency. When the reaction temperature is too low, the reaction tends to proceed too slowly. When the reaction temperature is too high, the side reactions such as polymerization between glycidol molecules tend to undesirably occur.
In addition, the reaction conducted under a solvent-free condition is excellent from the viewpoint of a good industrial convenience. However, when the reaction system is in a highly viscous state or non-uniform state, the reaction may be conducted in the presence of an adequate amount of a suitable solvent.

Examples of the solvent include amphipatic solvents such as tetrahydrofuran, dioxane and ethylene glycol dimethyl ether; hydrocarbon-based solvents, e.g., aliphatic hydrocarbons such as hexane, heptane, cyclohexane, methyl cyclohexane, isooctane and hydrogenated triisobutylene, and aromatic hydrocarbons such as benzene, toluene, xylene and ethyl benzene; and silicone-based solvents such as octamethyl cyclotetrasiloxane and decamethyl cyclopentasiloxane. Meanwhile, these solvents are preferably subjected to dehydration or deaeration upon use.

The alkyl (poly)glyceryl ether obtained in the process of the present invention is useful as a nonionic surfactant, and its applications as well as its configurations upon use are not particularly limited. For example, the alkyl (poly)glyceryl ether may be used in the form of a single compound, an aqueous solution, a water dispersion, an emulsion containing the other oil phase, a hydrous gel, an alcohol solution, a dispersion, or a mixture with solid substances such as an oil-based gel or waxes, as well as in a wet or infiltrated, or penetrated condition or configuration.
The alkyl (poly)glyceryl ether obtained in the process of the present invention may be extensively used, for example, as emulsifiers, solubilizers, dispersants, detergents, foaming agents, defoaming agents, penetrants, antibacterial agents, etc., for the purposes of emulsification, solubilization, dispersion, cleaning, foaming, defoaming, penetration, antibacterial effect, etc., in various applications such as food, cosmetics, toiletries, detergents, agricultural chemicals and drugs. In particular, the alkyl (poly)glyceryl ether obtained in the process of the present invention is excellent as a material for detergents.

### EXAMPLES

In the following Examples and Comparative Examples, the terms "%" and "ppm" represent "% by mass" and "ppm by mass", respectively.

### EXAMPLE 1

A 300 mL four-necked flask equipped with a stirrer, a nitrogen feed pipe and a thermometer was charged with 102.2 g of hexyl alcohol (available from Sigma-Aldrich Japan K.K.). At this time, the pH of the content in the flask was 6.7. Thereafter, while maintaining the reaction system in the flask at 150°C, 22.2 g of glycidol (available from Nacalai Tesque, Inc.; purified by distillation prior to the use) was dropped into the flask over 4 h (molar ratio of glycidol to alcohol (glycidol/alcohol) = 0.3). After completion of the dropping, the contents of the flask were reacted at 150°C over 58 h, thereby obtaining hexyl polyglyceryl ether. As a result of subjecting the obtained product to quantitative determination by gas chromatography, it was confirmed that the obtained hexyl polyglyceryl ether was composed of 41.0 g (83.7%) of hexyl glyceryl ether, 6.1 g (12.4%) of hexyl diglyceryl ether, 0.8 g (1.6%) of hexyl triglyceryl ether, and 2.3% of unknown components.

### COMPARATIVE EXAMPLE 1

A 300 mL four-necked flask equipped with a stirrer, a nitrogen feed pipe and a thermometer was charged with 102.2 g of hexyl alcohol and 0.35 g of potassium methoxide, followed by stirring the contents in the flask at 90°C over 2 h. At this time, the pH of the contents in the flask was 12.2. Thereafter, while maintaining the reaction system in the flask at 90°C, 7.4 g of glycidol (molar ratio of glycidol to alcohol (glycidol/alcohol) = 0.1) was equally divided into three parts, and the individual parts were intermittently added into the flask at intervals of 4 h. Then, the contents in the flask were reacted at 90°C for 2 h, thereby obtaining hexyl polyglyceryl ether. As a result of subjecting the obtained product to quantitative determination by gas chromatography, it was confirmed that the obtained hexyl polyglyceryl ether was composed of 6.8 g (49.4%) of hexyl glyceryl ether, 2.4 g (17.4%) of hexyl diglyceryl ether, 1.0 g (7.2%) of hexyl triglyceryl ether, and 26.0% of unknown components.

### COMPARATIVE EXAMPLE 2

A 300 mL four-necked flask equipped with a stirrer, a nitrogen feed pipe and a thermometer was charged with 102.2 g of hexyl alcohol and 0.14 g of boron trifluoride (47% ether solution). At this time, the pH of the contents in the flask was 2.0. While maintaining the reaction system in the flask at 50°C, 22.2 g of glycidol (molar ratio of glycidol to alcohol (glycidol/alcohol) = 0.3) was equally divided into three parts, and the individual parts were intermittently added into the flask at intervals of 4 h. Then, the contents in the flask were reacted at 50°C for 2 h, thereby obtaining hexyl polyglyceryl ether. As a result of subjecting the obtained product to quantitative determination by gas chromatography, it was confirmed that the obtained hexyl polyglyceryl ether was composed of 32.0 g (69.4%) of hexyl glyceryl ether, 10.1 g (21.9%) of hexyl diglyceryl ether, 2.9 g (6.3%) of hexyl triglyceryl ether, and 2.4% of unknown components.

**TABLE 1**

| | Monoglycerol compound | Diglycerol compound | Triglycerol compound | Unknown components |
|---|---|---|---|---|
| Example 1 | 83.7% | 12.4% | 1.6% | 2.3% |
| Comparative Example 1 | 49.4% | 17.4% | 7.2% | 26.0% |
| Comparative Example 2 | 69.4% | 21.9% | 6.3% | 2.4% |

From Table 1, it was confirmed that in Example 1, the monoglycerol compound was produced with a higher selectivity as compared to those obtained in Comparative Examples 1 and 2.

### INDUSTRIAL APPLICABILITY

In the production process of the present invention, a polyglyceryl ether, in particular, a monoglycerol compound, having a narrow molecular weight distribution can be produced with a high yield in a simplified manner while suppressing production of by-products such as polymers.

## Claims

1. A process for producing a (poly)glyceryl ether, comprising the step of reacting an alcohol with glycidol under a neutral condition.

2. The process according to claim 1, wherein the reaction is conducted at a temperature of from 100 to 200°C.

3. The process according to claim 1 or 2, wherein the glycidol is dropped at a rate of 1% by mass/min or less on the basis of a whole amount of the glycidol to be charged, to react with the alcohol.

4. The process according to any one of claims 1 to 3, wherein the alcohol is a compound represented by the following general formula (1):
R¹-(OR²)ₙO-H (1)
wherein R¹ is a hydrocarbon group having 4 to 24 carbon atoms; R² is an alkylene group having 2 or 3 carbon atoms; and n represents an average polymerization degree of the oxyalkylene group and is a number of from 0 to 20.

5. The process according to any one of claims 1 to 4, wherein the (poly)glyceryl ether is a compound represented by the following general formula (3):
R¹-(OR²)ₙO-(C₃H₅O₂)ₘ-H (3)
wherein R¹, R² and n are the same as defined above; and m represents an average polymerization degree of the glycerol residue and is a number of from 1 to 10.

6. A use of the (poly)glyceryl ether produced by the process as defined in any one of claims 1 to 5, for detergents.
